# FASCICULE DE BREVET EUROPEEN

(11) **EP 1 560 578 B1**
(45) Date de publication et mention de la délivrance du brevet: **07.06.2006**
(21) Numéro de dépôt: 03767891.9
(22) Date de dépôt: 04.11.2003
(51) Int. Cl.: A61K 31/381, A61P 1/04

(54) **UTILISATION DU SEL DISTRONTIQUE DE L'ACIDE 2-¬N,N-DI(CARBOXYMETHYL)AMINO|-3-CYANO-4-CARBOXYMETHYL-THIOPHÈNE-5-CARBOXYLIQUE POUR L'OBTENTION DE MÉDICAMENTS DESTINÉS AU TRAITEMENT DES DOULEURS GASTRO-DUODÉNALES**
VERWENDUNG EINES DISTRONTIUM SALZES DER 2-¬N,N-DI(CARBOXYMETHYL)AMINO|-3-CYANO-4-CARBOXYMETHYL-THIOPHEN-5-CARBONSÄURE ZUR HERSTELLUNG EINES ARZNEIMITTELS ZUR BEHANDLUNG VON GASTRODUODENALEN SCHMERZEN
USE OF THE DISTRONTIUM SALT OF THE ACID 2-¬N,N-DI(CARBOXYMETHYL)AMINO|-3-CYANO-4-CARBOXYMETHYL-THIOPHEN-5-CARBOXYLATE FOR THE PRODUCTION OF MEDICAMENTS FOR THE TREATMENT OF GASTRO-DUODENAL PAIN

(30) Priorité: 05.11.2002 FR 0213805
(43) Date de publication de la demande: 10.08.2005
(73) Titulaire: Les Laboratoires Servier, 92415 Courbevoie Cédex (FR)
(72) Inventeur: TSOUDEROS, Yannis, F-75014 Paris (FR)
(86) Numéro de dépôt international: PCT/FR2003/003279
(87) Numéro de publication internationale: WO 2004/043455

(56) Documents cités:
- EP-A- 0 813 869
- EP-B- 0 415 850
- US-A- 5 866 168
- US-A- 6 146 636
- US-A1- 2002 055 535

## Description

La présente invention concerne l'utilisation du sel distrontique de l'acide 2-[N,N-di(carboxyméthyl)amino]-3-cyano-4-carboxyméthyl-thiophène-5-carboxylique pour l'obtention de médicaments destinés au traitement des douleurs gastro-duodénales.

Le sel distrontique de l'acide 2-[N,N-di(carboxyméthyl)amino]-3-cyano-4-carboxyméthylthiophène-5-carboxylique a été décrit dans le brevet EP 0 415 850. Ses propriétés anti-ostéoporotiques permettent son utilisation des maladies osseuses comme l'ostéoporose. Il peut également être utilisé dans le traitement du vieillissement cutané et vasculaire, des affections hépatiques et des affections dentaires.

Le sel distrontique de l'acide 2-[N,N-di(carboxyméthyl)amino]-3-cyano-4-carboxyméthylthiophène-5-carboxylique possède d'autre part des propriétés antiarthrosiques qui le rendent utile dans le traitement de l'arthrose comme décrit dans le brevet EP 0 813 869.

L'utilisation de sels de strontium ou de dérivés de thiophène sous forme de sels distrontiques est également connu dans le traitement des maladies gastrointestinales (US-A-5866168, US-A-6146 636 et US-A-2002055535).

La demanderesse a présentement découvert que le sel distrontique de l'acide 2-[N,N-di(carboxyméthyl)amino]-3-cyano-4-carboxyméthyl-thiophène-5-carboxylique de formule (I) ainsi que ses hydrates possède des propriétés gastroprotectrices permettant son utilisation pour l'obtention de médicaments destinés au traitement des douleurs gastro-duodénales :

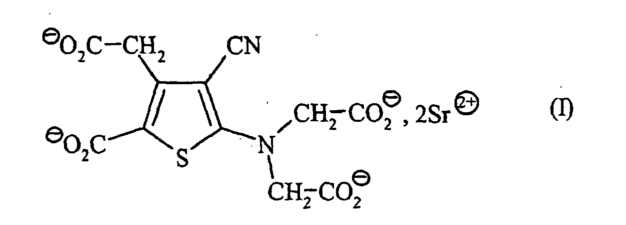

En effet, il a été montré dans une étude clinique que le composé de formule (I) dénommé également ranélate de strontium permettait d'améliorer significativement les douleurs gastriques.

Ce résultat est d'autant plus surprenant que lors d'une étude in vivo réalisée avec un autre sel de strontium (le chlorure de strontium), il a été observé que le chlorure de strontium provoquait chez des animaux traités des lésions superficielles hémorragiques très rapidement.

L'homme du métier n'aurait ainsi sans doute pas imaginé qu'un autre sel de strontium, en l'occurrence le ranélate de strontium permettrait d'obtenir l'effet inverse.

Ces résultats tout à fait surprenants permettent donc d'envisager l'utilisation du ranélate de strontium et de ses hydrates pour l'obtention de compositions pharmaceutiques utiles dans le traitement des douleurs gastro-duodénales et notamment des gastrites et des duodénites et de toutes les inflammations de la muqueuse de l'estomac qu'elles soient aiguës ou chroniques.

Les gastrites et duodénites correspondent à des états d'irritation de la muqueuse digestive qui s'accompagnent de douleurs abdominales quotidiennes rythmées par les repas. Elles sont aggravées par différents types d'aliments ou de boissons alcoolisées. Elles peuvent précéder la survenue d'ulcères. Elles justifient l'administration de traitements divers pour protéger la muqueuse, ou diminuer la sécrétion acide.

Les compositions pharmaceutiques seront présentées sous des formes convenant aux administrations par voie orale, parentérale, transcutanée, nasale, rectale, perlinguale, et notamment sous forme de préparations injectables, comprimés, comprimés sublinguaux, glossettes, gélules, capsules, tablettes, suppositoires, crèmes, pommades, gels dermiques, etc...

Outre le ranélate de strontium et ses hydrates éventuellement, les compositions pharmaceutiques selon l'invention contiennent un ou plusieurs excipients ou véhicules choisis parmi des diluants, des lubrifiants, des liants, des agents de désintégration, des absorbants, des colorants, des édulcorants, etc...

A titre d'exemple et de manière non limitative, on peut citer :
◆ *pour les diluants* : le lactose, le dextrose, le sucrose, le mannitol, le sorbitol, la cellulose, la glycérine,
◆ *pour les lubrifiants :* la silice, le talc, l'acide stéarique et ses sels de magnésium et de calcium, le polyéthylène glycol,
◆ *pour les liants :* le silicate d'aluminium et de magnésium, l'amidon, la gélatine, la tragacanthe, la méthylcellulose, la carboxyméthylcellulose de sodium et la polyvinylpyrrolidone,
*◆ pour les désintégrants* : l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne entre 25 mg et 6 g de ranélate de strontium par 24 heures, par exemple entre 100 mg et 4 g de produit par 24 heures.

De manière préférentielle, la dose journalière de ranélate de strontium sera de 2 g par jour.

### Etude clinique :

Cette étude a été réalisée sur 1649 patients. 719 ont été traités par le ranélate de strontium, 723 ont été traités par un placebo. La durée de traitement a été de 1101 ± 321 jours. La dose administrée de ranélate de strontium a été de 2 g/jour.

Il a été observé après traitement que les douleurs gastriques ont été significativement diminuées sur le groupe de patients traités par le ranélate de strontium (au moins 30%) comparativement au groupe de patients traités par le placebo.

## Revendications

1. Utilisation du sel distrontique de l'acide 2-[N,N-di(carboxyméthyl)amino]-3-cyano-4-carboxyméthyl-thiophène-5-carboxylique ou de ses hydrates pour l'obtention d'un médicament destiné au traitement des douleurs gastro-duodénales.

2. Utilisation selon la revendication 1 **caractérisée en ce que** le médicament est destiné au traitement des gastrites.

3. Utilisation selon la revendication 1 **caractérisée en ce que** le médicament est destiné au traitement des duodénites.

## Claims

1. Use of the distrontium salt of 2-[N,N-di(carboxymethyl)amino]-3-cyano-4-carboxymethyl-thiophene-5-carboxylic acid or hydrates thereof in obtaining a medicament intended for the treatment of gastro-duodenal pain.

2. Use according to claim 1, **characterised in that** the medicament is intended for the treatment of gastritis.

3. Use according to claim 1, **characterised in that** the medicament is intended for the treatment of duodenitis.

## Patentansprüche

1. Verwendung eines Distrontiumsalzes der 2-[N,N-Di(carboaymethyl)amino]-3-cyano-4-carboxymethylthiophen-5-carbonsäure oder deren Hydrate zur Herstellung eines Medikamentes zur Behandlung von gastroduodenalen Schmerzen.

2. Verwendung gemäß Anspruch 1, **dadurch gekennzeichnet, dass** das Medikament zur Behandlung von Magenschleimhautentzündungen/Gastritiden ist.

3. Verwendung gemäß Anspruch 1, **dadurch gekennzeichnet, dass** das Medikament zur Behandlung von Zwölffingerdarmentzündungen/Duodenitiden ist.
